(19) **Europäisches Patentamt**

**European Patent Office**

**Office européen des brevets**

(11) **EP 0 899 855 B1**

(12) **EUROPÄISCHE PATENTSCHRIFT**

(45) Veröffentlichungstag und Bekanntmachung des Hinweises auf die Patenterteilung:
**08.03.2006 Patentblatt 2006/10**

(51) Int Cl.:
*H02K 7/09* (2006.01)     *F16C 39/06* (2006.01)

(21) Anmeldenummer: **97810596.3**

(22) Anmeldetag: **25.08.1997**

(54) **Magnetgelagerte Rotationsanordnung**

Rotary device with magnetic bearing

Dispositif rotatif avec palier magnétique

(84) Benannte Vertragsstaaten:
**CH DE FR GB LI**

(43) Veröffentlichungstag der Anmeldung:
**03.03.1999 Patentblatt 1999/09**

(73) Patentinhaber: **Levitronix LLC**
**Waltham, MA 02451 (US)**

(72) Erfinder: **Schöb, Reto,Dr.**
**CH-8604 Volketswil (DE)**

(74) Vertreter: **Sulzer Management AG**
**KS/Patente/0007**
**Zürcherstrasse 12**
**8401 Winterthur (CH)**

(56) Entgegenhaltungen:
| | |
|---|---|
| **EP-A- 0 130 541** | **EP-A- 0 177 274** |
| **EP-A- 0 512 516** | **WO-A-96/31934** |
| **WO-A-97/15978** | **DE-A- 4 104 250** |
| **US-A- 4 381 875** | |

EP 0 899 855 B1

**Beschreibung**

Die Erfindung betrifft eine magnetgelagerte Rotationsanordnung.

Magnetgelagerte Rotationsanordnungen kommen heutzutage in vielen Anwendungsgebieten zum Einsatz, insbesondere aber dort, wo mechanisch gelagerte Rotationsanordnungen erhebliche Nachteile aufweisen, so z.B. bei Rührwerken oder Fördervorrichtungen für hochreine oder biologische Flüssigkeiten wie beispielsweise Blut. Auch bei Anwendungen wie Reinraumgebläsen oder Trägervorrichtungen für Wafer (sogenannte Wafer-Carrier), wo hohe Anforderungen an die Reinheit gestellt werden und es zu keinerlei Verunreinigungen kommen darf, wie sie z.B. durch Gase hervorgerufen werden, die aus Schmiermitteln von mechanischen Lagerungen entweichen können, kommen magnetgelagerte Rotationsanordnungen zum Einsatz. Bei diesen Anwendungen muss sowohl eine Lagerung des Rotors (der z.B. als Flügelrad einer Pumpe ausgebildet sein kann) als auch eine rotatorische Bewegung desselben möglich sein.

Aus der DE4104250 ist eine magnetgelagerte Rotationsanordnung mit einem scheiben- oder ringförmigen magnetisch gelagerten Rotor bekannt, der einen aus einem ferromagnetischen Material bestehenden Ring und Permanentmagnete aufweist, die in Umfangsrichtung verteilt auf dem scheiben- oder ringförmigen Rotor angeordnet sind. Die Permanentmagnete erzeugen einen Vormagnetisierungsfluss. Die magnetgelagerte Rotationsanordnung weist weiterhin einen Stator mit Mitteln zur Erzeugung eines mit dem Vormagnetisierungsfluss wechseMrirkenden Feldes auf, das eine Drehung des Rotors bewirkt. Die Mittel des Stators und die Permanetmagnete des Rotors bilden dabei einen etektronisch kommutierten Gleichstrommotor. Der Stator weist einen axial magnetisierten Permanentmagnetring auf, der zur magnetischen Lagerung mit dem Rotor zusammenwirkt Der Stator weist noch weitere Mittel auf, die zur Regelung der radialen Lage mit dem Rotor zusammenwirken.

In der EP-A-0,130,541 ist eine magnetgelagerte Rotationsanordnung mit einem Schwungrad beschrieben. Solche Vorrichtungen kommen beispielsweise bei der Steuerung der Orientierung von Satelliten zum Einsatz. Sowohl im Rotor wie auch im Stator sind dabei ringförmige Permanentmagneten für die magnetische Lagerung des Rotors vorgesehen, darüber hinaus umfasst der Stator noch Steuerwicklungen, um eventuelle Auslenkungen des Rotors aus der Sollage korrigieren zu können. Für den Antrieb des Rotors sind im Rotor und im Stator noch weitere, separate Antriebsmittel vorgesehen. Dies hat zur Folge, dass die gesamte Konstruktion vergleichsweise aufwendig wird.

Aufgabe der Erfindung ist es, eine magnetgelagerte Rotationsanordnung vorzuschlagen, mit welcher gleichzeitig eine magnetische Lagerung wie auch eine Drehung des Rotors bewirkt werden kann. Die magnetgelagerte Rotationsanordnung soll dabei möglichst wenig aufwendig sein und soll insbesondere auch die eingangs genannten Anwendungen gestatten können.

Erfindungsgemäss wird diese Aufgabe durch eine magnetgelagerte Rotationsanordnung gelöst, wie sie durch die Merkmale des Patentanspruchs 1 charakterisiert ist. Vorteilhafte Ausgestaltungen ergeben sich aus den Merkmalen der abhängigen Patentansprüche.

Im folgenden wird die Erfindung anhand der Zeichnung näher erläutert. Es zeigen, schematisch und/oder im Schnitt:

Fig. 1　Ausführungsbeispiele des Rotors der erfindungsgemässen Rotationsanordnung mit einseitig auf dem Rotor angeordneten, axial magnetisierten Permanentmagneten,

Fig. 2　Ausführungsbeispiele des Rotors der erfindungemässen Rotationsanordnung mit beidseitig auf dem Rotor angeordneten, axial magnetisierten Permanentmagneten,

Fig. 3　Ausführungsbeispiele des Rotors der erfindungsgemässen Rotationsanordnung mit einseitig auf dem Rotor angeordneten, radial magnetisierten Permanentmagneten,

Fig. 4　Ausführungsbeispiele des Rotors der erfindungemässen Rotationsanordnung mit beidseitig auf dem Rotor angeordneten, radial magnetisierten Permanentmagneten,

Fig. 5　ein Ausführungsbeispiel des Rotors mit beidseitig auf dem Rotor angeordneten, auf der einen Seite des Rotors axial magnetisierten, auf der anderen Seite radial magnetisierten Permanentmagneten,

Fig. 6　ein Ausführungsbeispiel des Rotors mit Vorsprüngen und Einbuchtungen zwischen diesen Vorsprüngen (Reluktanzläufer),

Fig. 7　Ausführungsbeispiele von möglichen Anordnungen der Permanentmagnete,

Fig. 8　ein Ausführungsbeispiel einer erfindungsgemässen Rotationsanordnung mit einem Lagerstator und zwei Motorstatoren in einer Explosionsdarstellung,

Fig. 9　das Ausführungsbeispiel gemäss Fig. 8 im Schnitt,

Fig. 10　ein Ausführungsbeispiel analog Fig. 8, im Schnitt, jedoch mit radial magnetisierten Permanentmagneten,

Fig. 11　ein Ausführungsbeispiel analog Fig. 8, im Schnitt, jedoch mit radial magnetisierten Permanentmagneten im Rotor und axial magnetisierten Permanentmagneten im Stator,

Fig. 12 eine Aufsicht auf den Rotor des Ausführungsbeispiels der Fig. 8,

Fig. 13 eine Darstellung eines Schnitts gemäss der Linie XIII-XIII in Fig. 8,

Fig. 14 ein weiteres Ausführungsbeispiel der erfindungsgemässen Rotationsanordnung (Segmentanordnung),

Fig. 15 eine Prinzipdarstellung der Anordnung der U-Kerne und der um diese herum gewickelten Motorwicklungen,

Fig. 16 ein Ausführungsbeispiel der erfindungsgemässen Rotationsanordnung analog Fig. 14, jedoch mit senkrecht zur Lagerebene angeordneten U-Kernen,

Fig. 17 einen Schnitt entlang der Linie XVII-XVII der Fig. 16,

Fig. 18 ein weiteres Ausführungsbeispiel der erfindungsgemässen Rotationsanordnung mit zwei ringförmigen Eisenrückschlüssen am Rotor, von denen einer mit Vorsprüngen und Einbuchtungen versehen ist,

Fig. 19 ein weiteres Ausführungsbeispiel der erfindungsgemässen Rotationsanordnung, bei welchem der gesamte Rotor Vorsprünge und Einbuchtungen aufweist und von einem magnetisch schlecht leitenden Ring umgeben ist,

Fig. 20 ein Ausführungsbeispiel des Rotors, bei welchem der Rotor ein U-förmiges Profil aufweist, teilweise mit Vorsprüngen und Einbuchtungen versehen ist und teilweise von einem magnetisch schlecht leitenden Ring umgeben ist,

Fig. 21 ein Ausführungsbeispiel der erfindungsgemässen Rotationsanordnung, bei welcher der Motorstator mit einer Scheibenläuferwicklung versehen ist,

Fig. 22 ein Ausführungsbeispiel der erfindungsgemässen Rotationsanordnung, bei welcher die Drehung des Rotors mit Hilfe eines Antriebs bewirkt wird, der permanentmagnetisch mit dem Rotor koppelbar ist,

Fig. 23 ein Ausführungsbeispiel der erfindungsgemässen Rotationsanordnung analog Fig. 22, bei welchem jedoch die permanentmagnetische Kopplung des Antriebs mittels radial magnetisierter Permanentmagneten bewirkt wird,

Fig. 24 eine Anwendung der erfindungsgemässen Rotationsanordnung in Form einer Pumpe, insbesondere für hochreine oder biologische Flüssigkeiten, speziell für Blut und

Fig. 25 die Anordnung gemäss Fig. 14 (Segmentanordnung), jedoch mit einer anderen Sensorik.

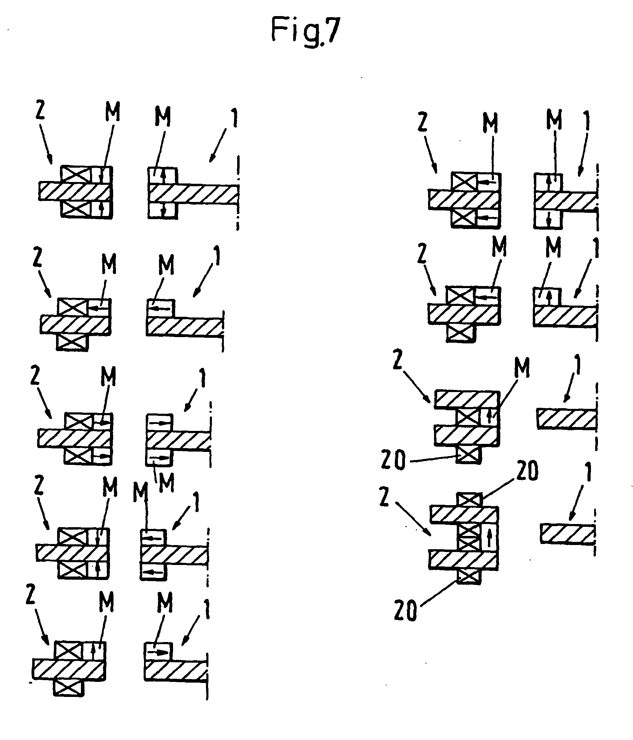

In der folgenden Figurenbeschreibung dienen die Ausführungen zu den Fig. 6, 18, 19 und 20, sowie die Figuren 6, 18, 19 und 20 selbst, lediglich dem besseren Verständnis und sind keine Ausführungsbeipiele gemäss der vorliegenden Erfindung. Das gleiche gilt für die Beschreibung und die Darstellungen der beiden letzten in Fig. 7 gezeigten Beispiele, die keinen Permanetmagneten M am Rotor 1 aufweisen.

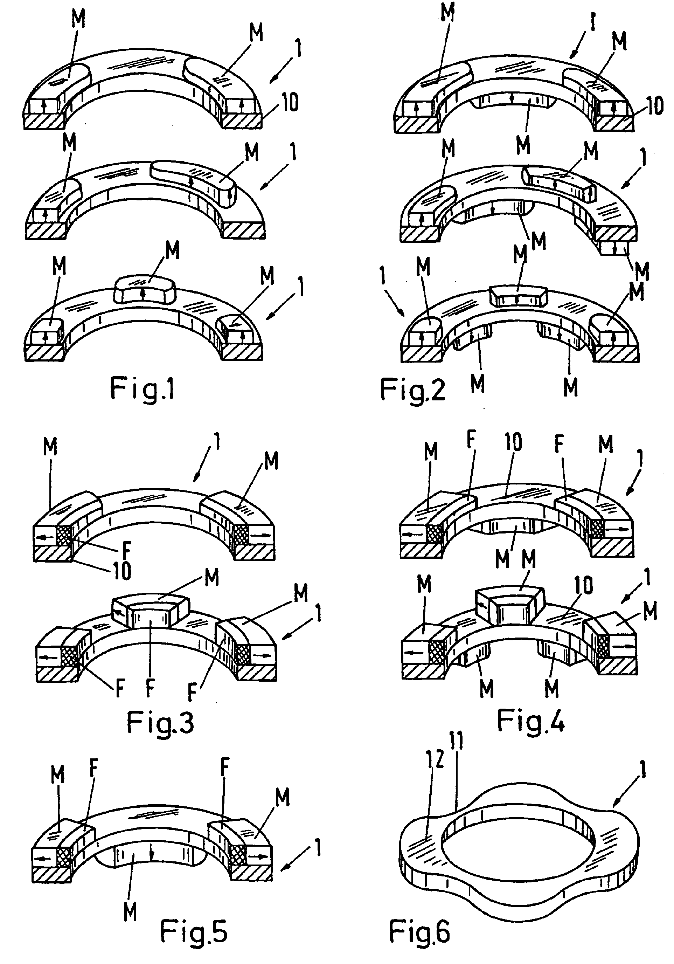

In Fig. 1 erkennt man drei Ausführungsbeispiele eines Rotors 1 der erfindungsgemässen Rotationsanordnung. Die drei Rotoren 1 umfassen jeweils einseitig des Rotors - auf der oberen Fläche eines aus ferromagnetischem Material bestehenden Rings 10 - angeordnete, axial magnetisierte, diskrete Permanentmagnete M. Diese Permanentmagnete erzeugen einen radial aus dem Ring 10 austretenden unipolaren (die Magnete auf dieser Seite des Rotors sind alle in gleicher Richtung magnetisiert) Vormagnetisierungsfluss. Im Bereich des Rings 10 (zwischen den Permanentmagneten M) ist der Vormagnetisierungsfluss in Umfangsrichtung betrachtet recht homogen. Insgesamt ist er jedoch in Umfangsrichtung betrachtet örtlich moduliert, weil zwischen den einzelnen diskreten Permanentmagneten M immer wieder Lücken vorgesehen sind. Durch die Wechselwirkung eines entsprechenden Dreh- oder Wechselfelds mit dem örtlich modulierten unipolaren Vormagnetisierungsfluss wird im Rotor 1 ein Drehmoment erzeugt. Die drei in Fig. 1 gezeigten Rotoren unterscheiden sich im wesentlichen durch die Anzahl der Pole bzw. Polpaare. Der oberste Rotor weist genau vier Pole (zwei Polpaare) auf, während der mittlere Rotor sechs Pole (drei Polpaare) und der untere acht Pole (vier Polpaare) aufweist.

Die in Fig. 2 gezeigten Ausführungsbeispiele des Rotors 1 weisen beidseitig des Rotors (also auf der oberen wie auf der unteren Fläche des Rotors) angeordnete, axial magnetisierte, Permanentmagnete M auf, wobei die Magnetisierungsrichtung der Permanentmagnete M auf der oberen Fläche des Rotors der Magnetisierungsrichtung der Permanentmagnete M auf der unteren Fläche des Rotors entgegengesetzt (sowohl auf der oberen als auch auf der unteren Fläche jedoch stets gleich gerichtet, also unipolar) ist.

Fig. 3 zeigt Ausführungsbeispiele des Rotors 1, bei denen wieder nur einseitig auf dem Rotor Permanentmagnete M angeordnet sind, während bei den Ausführungsbeispielen des Rotors gemäss Fig. 4 beidseitig auf dem Rotor Permanentmagnete angeordnet sind. Im Unterschied zu den Ausführungsbeispielen gemäss Fig. 1 und Fig. 2 sind die Permanentmagnete M aber radial magnetisiert. Ausserdem sind zur besseren Führung des magnetischen Flusses zusätzliche ferromagnetische Elemente F (z.B. aus Eisen) vorgesehen.

Fig. 5 zeigt ein Ausführungsbeispiel des Rotors 1, bei welchem beidseitig auf dem Rotor Permanentmagnete M angeordnet sind, auf der einen Seite (hier: unten) des Rotors axial magnetisierte Permanentmagnete, auf der anderen Seite des Rotors (hier: oben) radial magnetisierte Permanentmagnete. Fig. 6 zeigt ein Ausführungsbeispiel des Rotors mit Vorsprüngen 12 und Einbuchtungen 11, bei welchem die Modulation des unipolaren Vormagnetisierungsflusses also durch weichmagnetische Anisotropie bewirkt wird. Dieser Typ Rotor ist auch als sogenannter Reluktanzläufer bekannt.

In Fig. 7 sind mehrere Ausführungsbeispiele von möglichen Anordnungen der Permanentmagnete dargestellt. Dabei erkennt man, beginnend in der linken Spalte von oben nach unten und anschliessend in der rechten Spalte von oben nach unten:

- axial magnetisierte Permanentmagnete M beidseitig auf dem Rotor 1 als auch im Stator 2
- radial magnetisierte Permanentmagnete M einseitig auf dem Rotor 1 als auch im Stator 2
- radial magnetisierte Permanentmagnete M beidseitig auf dem Rotor 1 als auch im Stator 2
- radial magnetisierte Permanentmagnete M beidseitig auf dem Rotor 1 und axial magnetisierte Permanentmagnete M beidseitig im Stator 2
- radial magnetisierte Permanentmagnete M einseitig auf dem Rotor 1 und axial magnetisierte Permanentmagnete M einseitig im Stator 2
- axial magnetisierte Permanentmagnete M beidseitig auf dem Rotor 1 und radial magnetisierte Permanentmagnete M beidseitig im Stator 2
- axial magnetisierte Permanentmagnete M einseitig auf dem Rotor 1 und radial magnetisierte Permanentmagnete M einseitig im Stator 2
- axial magnetisierte Permanentmagnete M im Stator 2 und keine Permanentmagnete auf dem Rotor 1 (Reluktanzläufer), nur eine Steuerwicklung 20 im Stator 2
- axial magnetisierte Permanentmagnete M im Stator 2 und keine Permanentmagnete auf dem Rotor 1 (Reluktanzläufer), zwei Steuerwicklungen 20 im Stator 2
- axial magnetisierte Permanentmagnete M im Stator 2 und im Rotor 1, zwei Steuerwicklungen im Stator 2.. Grundsätzlich können die hier gezeigten Magnetisierungen auch umgekehrt orientiert sein.

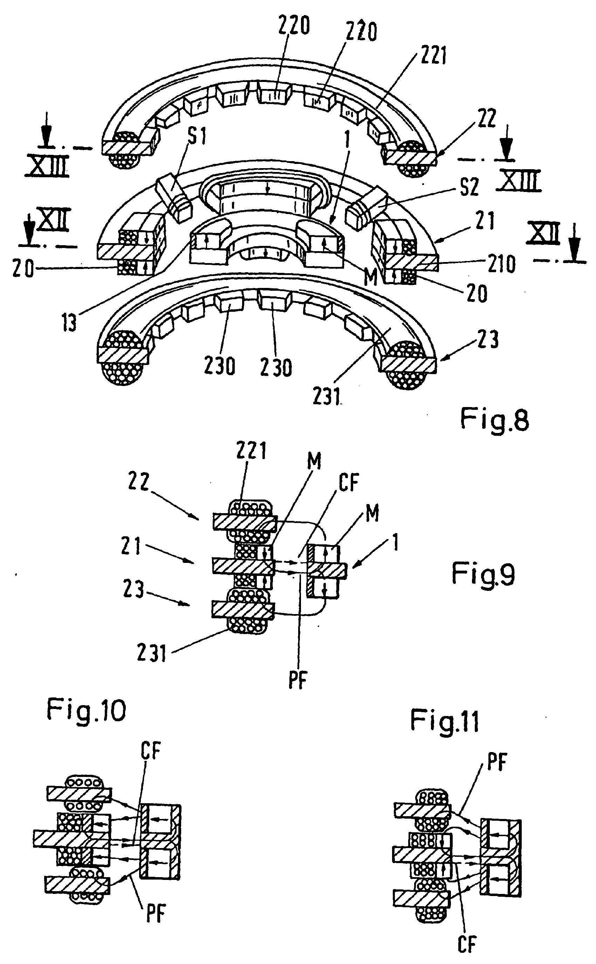

In Fig. 8 ist ein Ausführungsbeispiel einer erfindungsgemässen Rotationsanordnung in einer Explosionsdarstellung zu erkennen. Bei diesem Ausführungsbeispiel umfasst der Stator neben einem ringförmigen Lagerstator 21, der die magnetische Lagerung des Rotors 1 in einer Lagerebene bewirkt, noch zwei Motorstatoren 22 und 23, von denen der eine Motorstator 22 in einer ersten Motorebene parallel zur Lagerebene auf der einen Seite (hier: oberhalb) des Lagerstators 21 und der andere Motorstator 23 in einer zweiten Motorebene parallel zur Lagerebene auf der anderen Seite (hier: unterhalb) des Lagerstators 21 angeordnet ist.

Betrachtet man die Lagerebene, so erkennt man, dass der Rotor 1 beidseitig des Rotors angeordnete, axial magnetisierte, Permanentmagnete M aufweist. Ferner ist am Rotor 1 ein Sensorring 13 vorgesehen aus einem magnetisch schlecht leitenden, elektrisch jedoch gut leitenden Material (z.B. Aluminium), in welchem im Betrieb Wirbelströme auftreten, die wiederum Magnetfelder erzeugen, welche mit Hilfe von Sensoren S1,S2 im Lagerstator 21 ausgewertet werden. Auf diese (an sich bekannte) Weise kann die Position des Rotors 1 zu jedem Zeitpunkt genau bestimmt und nötigenfalls korrigiert werden.

Der ringförmige Lagerstator 21 weist in radialer Richtung auf den Rotor 1 zuweisende Zähne 210 auf, auf denen einerseits die statorseitigen Permanentmagnete M angeordnet sind und andererseits die Steuerwicklungen 20. Mit Hilfe der Steuerwicklungen kann der Permanentmagnetfluss selektiv verstärkt oder geschwächt werden, sodass bei einer Abweichung des Rotors 1 aus der Sollage der Rotor 1 wieder in die Sollage zurück bewegt werden kann.

Die beiden ringförmigen Motorstatoren 22 und 23 weisen ebenfalls in radialer Richtung nach innen weisende Zähne 220 bzw. 230 auf. Ferner sind sie jeweils mit einer Drehfeldwicklung 221 bzw. 231 versehen, mit der ein entsprechendes Drehfeld erzeugt werden kann, um den Rotor 1 rotatorisch anzutreiben. Solche Drehfeldwicklungen 221 bzw. 231 sind an sich bekannt - sie sind daher in Fig. 8 nur schematisch dargestellt. Da die Polzahl bzw. Polpaarzahl von Rotor und Stator übereinstimmen muss, und der Rotor eine Polzahl von vier (zwei Polpaare) beidseitig aufweist, müssen auch die Drehfeldwicklungen so ausgebildet sein, dass ein vierpoliges Drehfeld im Motorstator erzeugt wird. Natürlich ist es auch möglich einen der beiden Motorstatoren wegzulassen, wodurch sich allerdings das Drehmoment halbiert. In diesem Fall ist es auch sinnvoll, die Permanentmagnete auf der entsprechenden Seite des Rotors wegzulassen. Auf diese Weise lässt sich die Bauhöhe der Anordnung verringern.

Fig. 9 zeigt das Ausführungsbeispiel der Fig. 8 im Schnitt. Man erkennt in Fig. 9 einerseits den Verlauf des von den Permanentmagneten M erzeugten magnetischen Flusses (durchgezogene Linie PF), der von den Permanentmagneten M auf dem Rotor 1 ausgeht, über den Lufspalt in den (aus ferromagentischem Material bestehenden) Motorstator 22 bzw. 23 führt, von dort aus über die Permanentmagneten M im Lagerstator 21 weiterführt, durch das ferromagnetische Material (z.B. Eisen) des Lagerstators 21 und dessen Zähne 210 über den Luftspalt zwischen Lagerstator 21 und Rotor 1 zurück in das ferromagnetische Material (z.B. Eisen) des Rotors 1 führt, und sich schliesslich wieder in den Permanentmagneten M des Rotors 1 schliesst. Ein Teil des permanentmagnetischen Flusses geht auch direkt von den Permanentmagneten M auf dem Rotor 1 in die Permanentmagneten M auf dem Lagerstator 21, was in Fig. 9 nicht dargestellt ist.

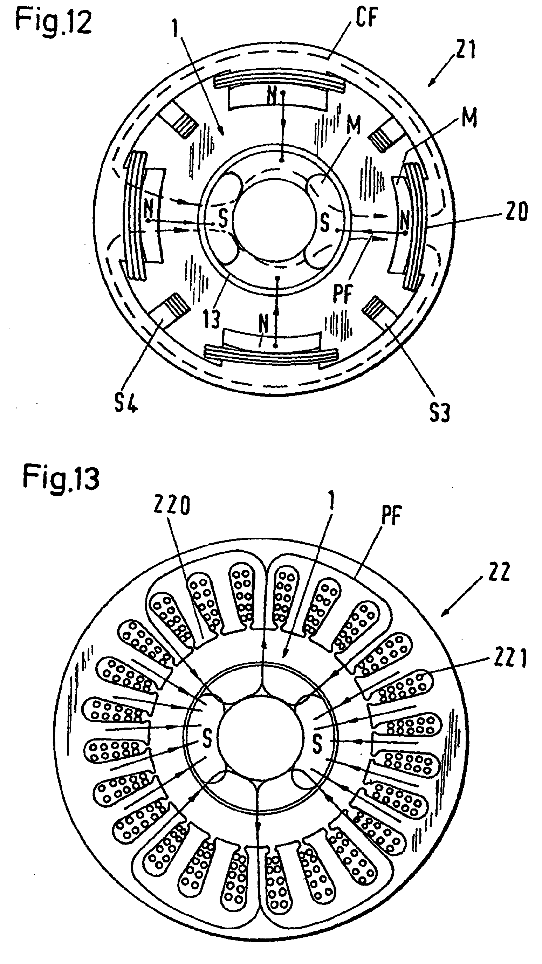

Ferner erkennt man in Fig. 9 im Luftspalt zwischen dem Lagerstator 21 und dem Rotor 1 noch den mit Hilfe der Steuerwicklungen 20 erzeugbaren Steuerfluss (gestrichelte Linie CF) zur Korrektur von Abweichungen des Rotors aus der Sollage. Der geschlossene magnetische Kreis des Steuerflusses ist in Fig. 9 nicht erkennbar, da er in der Lagerebene verläuft und somit prinzipiell in einer Ebene senkrecht zur Ebene des permanentmagnetischen Flusses verläuft (siehe hierzu Fig. 12). Lediglich im Bereich der Zähne 210 und im Luftspalt zwischen Lagerstator 21 und Rotor 1 (und natürlich auch beim Eintritt in den Rotor) kann eine Verstärkung oder Schwächung des permanentmagnetischen Flusses bewirkt werden, weil hier die beiden ansonsten sich nicht beeinflussenden Felder Komponenten gleicher Orientierung aufweisen. Fig. 10 und Fig. 11 zeigen jeweils Anordnungen analog zu Fig. 8 in einer Darstellung analog zu Fig. 9. Im Unterschied zu Fig. 8 und Fig. 9 sind jedoch die Permanentmagnete M im Rotor 1 und im Lagerstator 21 in Fig. 10 beide radial magnetisiert, in Fig. 11 sind die Permanentmagnete M im Rotor 1 radial magnetisiert, während sie im Lagerstator 21 axial magnetisiert sind.

Fig. 12 zeigt eine Aufsicht auf den Rotor gemäss dem Ausführungsbeispiel von Fig. 8, also eine Aufsicht auf die Lagerebene. In dieser Darstellung erkennt man nun besser den Verlauf des Steuerflusses CF (gestrichelte Linie), der mit Hilfe der Steuerwicklungen 20 erzeugbar ist, um den Rotor 1 bei einer Abweichung aus seiner Sollage wieder in die Sollage zurück bewegen zu können. Es ist klar, dass ein Teil des Steuerflusses auch über die beiden anderen Statorzähne rückgeschlossen ist, was hier aus Gründen der besseren Übersichtlichkeit jedoch nicht dargestellt ist. Ferner kommt natürlich bei dieser Darstellung der Verlauf des permanentmagnetischen Flusses PF (durchgezogene Linie) nicht besonders zur Geltung, zum Verlauf des permanentmagnetischen Flusses siehe Fig. 9. Schliesslich erkennt man in Fig. 12 noch die beiden zusätzlichen Sensoren S3 und S4, die in der Darstellung gemäss Fig. 8 nicht zu erkennen sind.

Fig. 13 zeigt einen Schnitt gemäss der Linie XIII-XIII in Fig. 8, also einen Schnitt in der ersten Motorebene. Man erkennt hier den Motorstator 22 und den Verlauf des von den Permanentmagneten M erzeugten permanentmagnetischen Flusses. Ferner erkennt man auch die in die einzelnen Nuten des Motorstators 22 eingelegte - hier vierpolige - Drehfeldwicklung 221 (das Drehfeld selbst ist jedoch nicht dargestellt). Wie eine solche Drehfeldwicklung ausgestaltet sein kann, ist dem Fachmann

hinreichend bekannt und wird daher hier nicht näher erläutert.

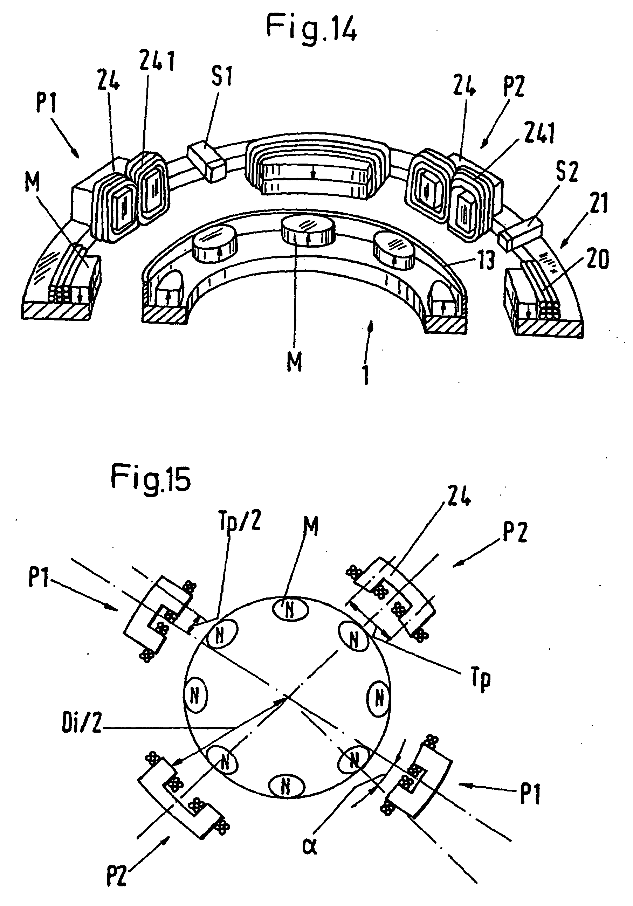

In Fig. 14 ist ein weiteres Ausführungsbeispiel der erfindungsgemässen Rotationsanordnung gezeigt, es handelt sich hierbei um eine Segmentanordnung. Diese Bezeichnung rührt daher, dass in den einzelnen Segmenten zwischen den den Permanentmagneten M im Lagerstator 21 auch die Mittel zur Erzeugung des Drehfelds (bzw. Wechselfelds) angeordnet sind, nämlich in Form von U-förmigen Spulenkernen 24 (z.B. Eisenkerne), auf welche Wicklungen 241 gewickelt sind, mit denen sich ein Drehfeld (bzw. ein Wechselfeld) erzeugen lässt. Die U-förmigen Spulenkerne 24 sind dabei quasi in der Lagerebene angeordnet. Der Vorteil ist eine geringe Bauhöhe. Man erkennt, dass es sich bei dem in Fig. 14 gezeigten Ausführungsbeispiel um einen Rotor mit der Polzahl sechzehn (acht Polpaare) handelt. Bei der hier dargestellten Anordnung handelt es sich um eine zweiphasige Anordnung, die vom Prinzip her 2,4,6,8,...usw. U-förmige Spulenkerne 24 aufweisen kann, hier weist sie vier Spulenkerne 24 auf, von denen zwei dargestellt sind (wegen der Halbschnittdarstellung).

In Fig. 15 ist die Anordnung der U-förmigen Spulenkerne 24 und die um diese Spulenkerne 24 herum gewickelten Wicklungen 241 in einer Prinzipdarstellung zu erkennen. Die Wicklungen von einander gegenüber liegenden Spulenkernen werden jeweils von dem gleichen Strom durchflossen, sie können in Reihe oder parallel geschaltet sein. Die beiden Phasen P1 und P2 (es handelt sich um eine zweiphasige Anordnung) sind elektrisch um 90° gegeneinander versetzt. Die Polteilung Tp beträgt

$$Tp = (\pi \cdot Di)/2p$$

wobei Di den Innendurchmesser eines Kreises bezeichnet, der die dem Rotor 1 zugewandten Pole der U-förmigen Spulenkerne 24 berührt, Di/2 also den Radius dieses Kreises darstellt (siehe Fig. 15), und p die Polpaarzahl.

Die Permanentmagnete M auf dem Rotor 1 folgen jeweils um einen Winkel von 45° versetzt aufeinander (es sind acht Permanentmagnete M, die gleichmässig über einen Winkel von 360° verteilt sind). Würde man nun die vier U-förmigen Spulenkerne 24 ebenfalls in der gleichen Regelmässigkeit über den Winkel von 360° verteilt (also um einen geometrischen Winkel von 90° versetzt zueinander) anordnen, so käme jeweils genau gleichzeitig ein Permanentmagnet M des Rotors zwischen vor den U-förmigen Spulenkernen zu liegen. Völlig unabhängig von dem elektrischen Versatzwinkel der beiden Phasen P1 und P2 kann auf diese Weise kein Anfahren des Rotors 1 erfolgen, weil bei einem Anwachsen und Abfallen des magnetischen Felds der jeweilige Permanentmagnet auf dem Rotor 1 lediglich stärker oder schwächer von dem jeweiligen U-förmigen Spulenkern 24 angezogen bzw. abgestossen würde. Man müsste den Rotor also zunächst (z.B. mechanisch) anstossen, um ihn in Drehung versetzen zu können.

Damit nun auch ein elektrisches Anfahren des Rotors 1 möglich ist, sind die beiden Phasen P1 und P2 (die elektrisch ja um 90° versetzt sind) geometrisch gegeneinander versetzt, und zwar idealerweise so, dass ein Permanentmagnet vollständig vor dem Spulenkern der einen Phase zu liegen kommt, wenn ein anderer Permanentmagnet vor dem Spulenkern der anderen Phase genau zur Hälfte zu liegen kommt, sodass mit einer der beiden Phasen P1 oder P2 immer ein auf den Rotor 1 wirkendes Drehmoment erzeugt werden kann. Dieser geometrische Versatz beträgt

$$(360°/2p) \cdot ((1/m) + k),$$

wobei p für die Polpaarzahl steht (hier also 8), m die Anzahl Phasen bezeichnet (hier 2) und k eine natürliche Zahl bedeutet, hier also prinzipiell der Versatzwinkel $11.25° + k \cdot 22.5°$ beträgt; speziell bei dem gezeigten Ausführungsbeispiel gilt k = 4. Man erkennt in Fig. 15 den Winkel $\alpha$ = 11.25° und den entsprechenden Versatz der Spulenkerne 24. Analog zu diesem Beispiel sind natürlich Anordnungen mit beliebigen Phasenzahlen, insbesondere auch ein- und dreiphasige Anordnungen wie auch beliebige Polpaarzahlen möglich. Auch ist es denkbar, Motorsegmente mit mehreren Nuten pro Polteilung und mit einer näherungsweise sinusförmigen Durchflutungsverteilung einzusetzen. Ebenso sind Luftspaltwicklungen denkbar, um nach Möglichkeit Nutrastmomente zu verhindern.

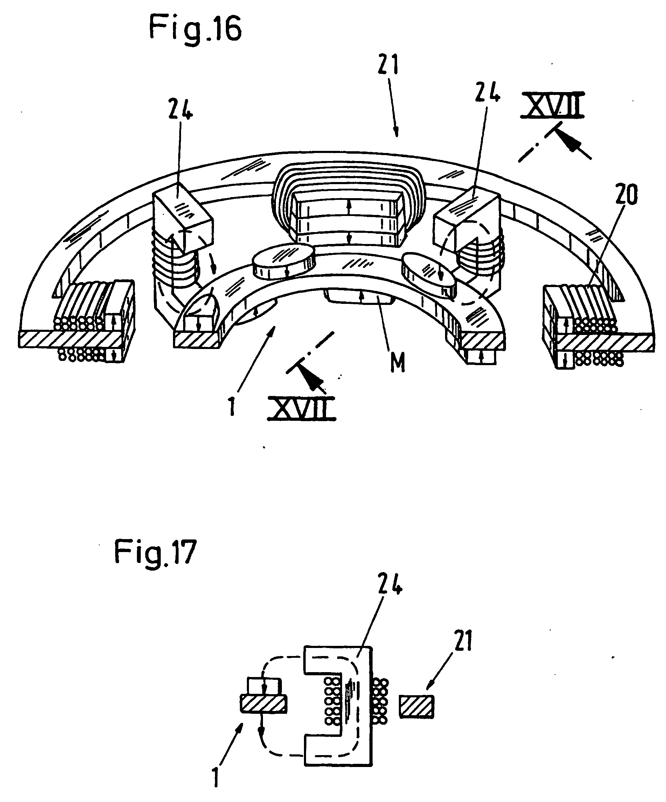

Fig. 16 zeigt ein weiteres Ausführungsbeispiel der erfindungsgemässen Rotationsanordnung, welches vom grundsätzlichen Prinzip her dem Ausführungsbeispiel gemäss Fig. 14 entspricht, bei welchem allerdings die Spulenkerne 24 senkrecht zur Lagerebene angeordnet sind. Darüberhinaus sind auch Permanentmagnete M beidseitig des Rotors 1 angeordnet, entsprechendes gilt auch für den Lagerstator 21. Fig. 17 zeigt einen entsprechenden Schnitt entlang der Linie XVII-XVII in Fig. 16. Eine solche Anordnung entspricht einem sogenannten "Transveralsflussmotor", hier als Segmentmotor mit gesplitteter Phase gezeigt.

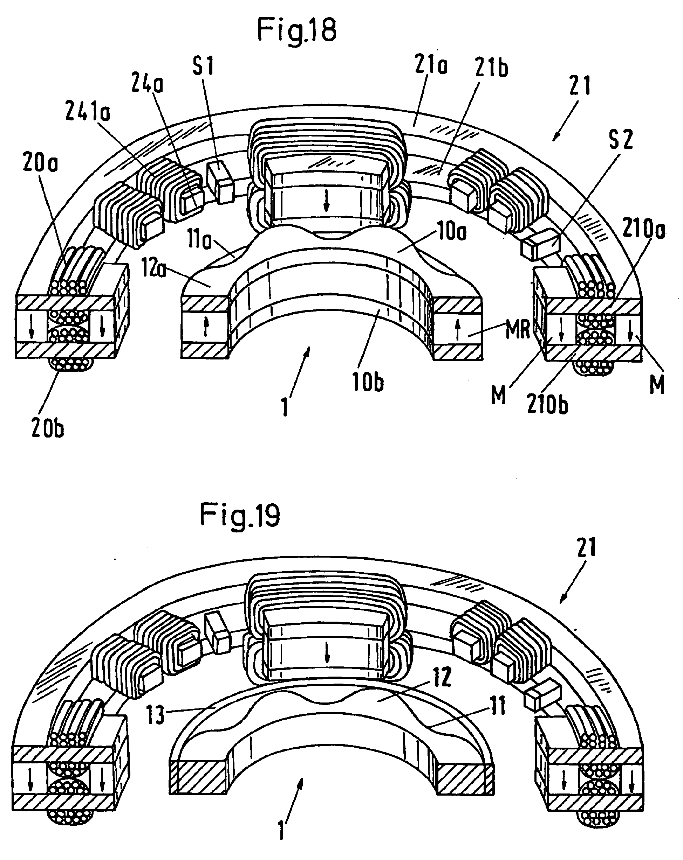

Fig. 18 zeigt ein weiteres Ausführungsbeispiel der erfindungsgemässen Rotationsanordnung. Bei diesem Ausführungsbeispiel umfasst der Rotor 1 zwei aus einem ferromagnetischen Material bestehende Ringe 10a und 10b, die Vorsprünge 12a und Einbuchtungen 11a (bzw. 12b und 11b, die nicht zu erkennen sind) aufweist (Reluktanzläufer). Zwischen den beiden Ringen 10a und 10b ist ein axial magnetisierter Permanentmagnetring MR angeordnet, der nicht strukturiert ist. Der ringförmigen Lagerstator 21 umfasst ebenfalls zwei Ringe 21a und 21b, wobei zwischen den Zähnen 210a und 210b der beiden Ringe axial magnetisierte Permanentmagnete M angeordnet sind. Ferner erkennt man noch die Steuerwicklungen 20a und 20b (im Prinzip ist nur eine Steuerwicklung nötig), sowie die Sensoren S1 und S2 zur Bestimmung der jeweils aktuellen Position des Rotors. Die Mittel zum Antrieb des Rotors 1 sind in Form von Spulenkernen 24a am oberen Ring 21a des Lagerstators 21 angeformt, wobei um die Spulenkerne 24a herum entsprechende Wicklungen 241a gewickelt sind. Dieses Ausführungsbeispiel zeichnet sich durch einen hohen permanentmagnetischen Vormagnetisierungsfluss aus, was es ermöglicht, den Luftspalt zwischen Stator und Rotor für bestimmte Anwendungen zu vergrössern und dennoch eine zuverlässige magnetische Lagerung zu erreichen.

Das Ausführungsbeispiel gemäss Fig. 19 unterscheidet sich von dem Ausführungsbeispiel gemäss Fig. 18 im wesentlichen durch die andere Ausgestaltung des Rotors 1. Dieser umfasst keine Permanentmagnete, sondern ist aus einem ferromagnetischen Material, weist die besagte Struktur mit den Vorsprüngen 12 und den Einbuchtungen 11 auf und ist von einem Sensorring 13 aus einem magnetisch schlecht leitenden, jedoch elektrisch gut leitenden Material (z.B. aus Aluminium) umgeben. Dieses Ausführungsbeispiel ist insbesondere für solche Anwendungen vorteilhaft, bei denen der Rotor 1 nach einer Anwendung weggeworfen bzw. rezykliert werden muss, weil die entsprechende Anwendung dies gebietet (z.B. beim Fördern von Blut). Der Rotor 1 ist nämlich wegen der nicht vorhandenen Permanentmagnete vergleichsweise einfach und kostengünstig in der Herstellung.

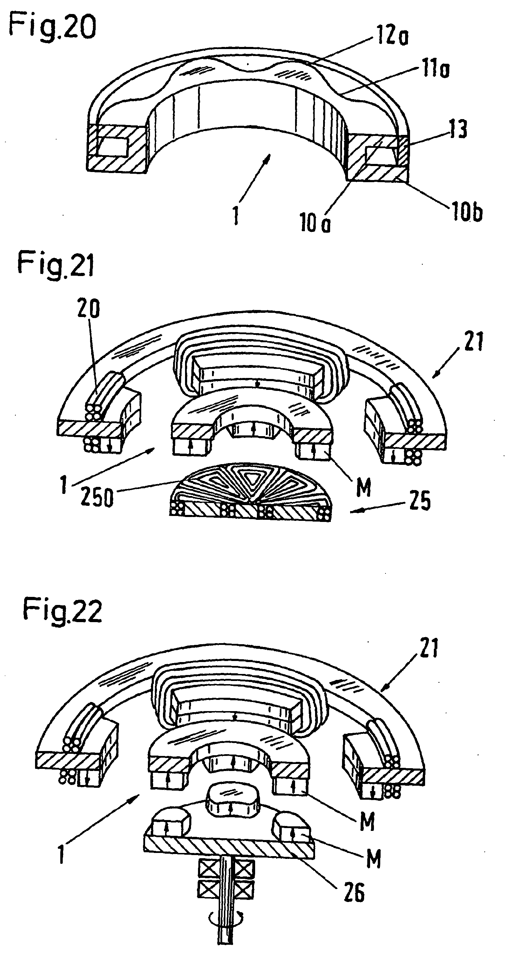

Fig. 20 zeigt ein Ausführungsbeispiel eines Rotors 1, der aus einem ferromagnetischen Material hergestellt ist und ein U-förmiges Profil aufweist. Er ist von einem Sensorring 13 umgeben. Durch das U-förmige Profil weist der Rotor 1 quasi einen oberen Ring 10a und einen unteren Ring 10b auf, die über den Steg miteinander verbunden sind. Während der obere Ring 10a eine Struktur mit Vorsprüngen 12a und Einbuchtungen 11a aufweist, weist der untere Ring 10b keine Struktur auf. Ein derartiger Rotor ist insofern vorteilhaft, als die Rüttelkräfte, die aufgrund der Strukturierung auftreten, reduziert werden, da der untere Ring 10b keine Struktur aufweist. Zudem ist es möglich, mit Hilfe von Hall-Sensoren im Luftspalt des unteren Rings die Position des Rotors zu messen. Ansonsten entsprechen die Vorteile denen des Rotors gemäss Fig. 19. Ausserdem sind die Reluktanzkräfte hinsichtlich einer axialen Lagerung des Rotors grösser (wegen der Nut zwischen den einzelnen "Ringen" des U-förmigen Profils).

Fig. 21 zeigt ein Ausführungsbeispiel der erfindungsgemässen Rotationsanordnung mit einem Lagerstator 21 der bereits beschriebenen Art mit einseitig angeordneten, axial magnetisierten, Permanentmagneten M, sowie mit einem Motorstator 25 mit einer Scheibenläuferwicklung 250. Eine Scheibenläuferwicklung ist eine quasi in einer Ebene angeordnete Flachwicklung zur Erzeugung eines Drehfelds. Scheibenläuferwicklungen sind an sich bekannt, sie können insbesondere eine Multilayerstruktur aufweisen. Die Scheibenläuferwicklung ist vorzugsweise eisenlos, wodurch axiale Zugkräfte auf den Rotor 1 praktisch völlig vermieden werden.

Fig. 22 zeigt ein Ausführungsbeispiel der erfindungsgemässen Rotationsanordnung, bei welchem die Drehung des Rotors 1 mit Hilfe eines Antriebs bewirkt wird, der permanentmagnetisch mit dem Rotor 1 koppelbar ist. Lagerstator 21 und Rotor 1 entsprechen dabei dem Lagerstator 21 und dem Rotor 1 der Fig. 21. Einzig der Antrieb ist unterschiedlich ausgebildet, er umfasst nämlich hier eine aus ferromagnetischem Material bestehende Scheibe 26, auf der axial magnetisierte Permanentmagnete M angeordnet sind, wobei die Anordnung der Permanentmagnete M auf der Scheibe 26 der Anordnung der Permanentmagnete M auf dem Rotor 1 entspricht. Die Scheibe 26 ist antreibbar mechanisch gelagert. Über die permanentmagnetische Kopplung (hier: permanentmagnetische Axialkopplung) folgt beim Antreiben der Scheibe 26 der Rotor 1 aufgrund von Reluktanzkräften der Drehung der Scheibe 26 synchron.

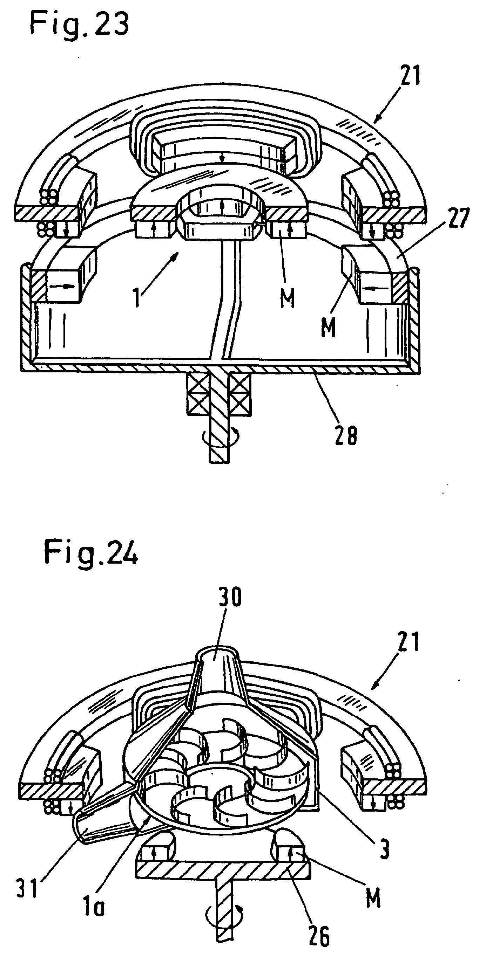

Das Ausführungsbeispiel gemäss Fig. 23 unterscheidet sich von dem Ausführungsbeispiel gemäss Fig. 22 lediglich dadurch, dass die Permanentmagnete M des Antriebs nicht auf einer ferromagnetischen Scheibe, sondern an einem ferromagnetischen Ring 27 befestigt sind, der seinerseits mit einem Gestänge 28 verbunden ist, welches antreibbar mechanisch gelagert ist. Das Funktionsprinzip ist jedoch gleich: Aufgrund von Reluktanzkräften folgt der Rotor 1 bei einer Drehung des Gestänges synchron.

Fig. 24 zeigt schliesslich eine Anwendung der erfindungsgemässen Rotationsanordnung in Form einer Pumpe, insbesondere für hochreine oder biologische Flüssigkeiten, speziell für Blut. Der Lagerstator 21 und der Antrieb 26 entsprechen dabei den entsprechenden Teilen des Ausführungsbeispiels gemäss Fig. 22. Der Rotor ist hier als Flügelrad 1a ausgebildet und ist in einem abgeschlossenen Pumpengehäuse 3 aus einem magnetisch schlecht leitenden Material (z.B. Kunststoff oder Chromstahl) angeordnet. Das Pumpengehäuse 3 weist zwei Anschlussstutzen 30 und 31 auf zum Zuführen bzw. Abführen der zu fördernden Flüssigkeit beispielsweise Blut. Eine solche Pumpe muss nach dem einmaligen Gebrauch regelmässig entsorgt werden. Dies kann auf einfache Weise erfolgen, indem die Zuführleitung und die Abführleitung von den Anschlussstutzen 30 bzw. 31 abgezogen wird und ein neues Pumpengehäuse eingesetzt wird. Die übrige Vorrichtung kann jedoch wiederverwendet werden, darüberhinaus ist der Austausch des Pumpengehäuses einfach und komfortabel durchzuführen.

Ferner soll noch erwähnt werden, dass bei der erfindungsgemässen Rotationsanordnung, insbesondere hinsichtlich ihrer verwendung als Blutpumpe, etliche Aspekte aus der WO-A-96/31934 ebenfalls denkbar sind. So ist sowohl eine Innenläufer- wie auch eine Aussenläuferanordnung denkbar. Weiterhin kann, wie bereits erwähnt, zwischen dem Stator und dem Rotor eine hermetische Trennstelle (wie z.B. ein Spaltrohr oder ein Gefäss) angeordnet sein. Der Rotor selbst kann mit einem Kunststoff, mit einer Keramik oder mit einem anderen nicht metallischen Werkstoff eingekapselt werden. Ferner kann, wie teilweise auch beschrieben, ein um den Rotor herum angeordneter Ring aus elektrisch gut leitfähigem aber magnetisch schlecht leitfähigem Material vorgesehen sein, der als Sensorring für die Positionsmessung mittels Wirbelstromsensoren dienen kann. Die Bestimmung der jeweiligen Winkelposition des Rotors kann mittels Hallsensoren erfolgen, die ebenfalls als Positionssensoren dienen können, was vorteilhaft bei der Messung durch ein elektrisch leitfähiges Spaltrohr hindurch ist. Der Rotor kann zusammen mit dem ihn umgebenden Gefäss (siehe z.B. die in anhand von Fig. 24 beschriebene Blutpumpe) austauschbar und wegwerfbar bzw. rezyklierbar sein, um die Sterilität garantieren zu können. Näheres zu all diesen Dingen ist der WO-A-31394 zu entnehmen.

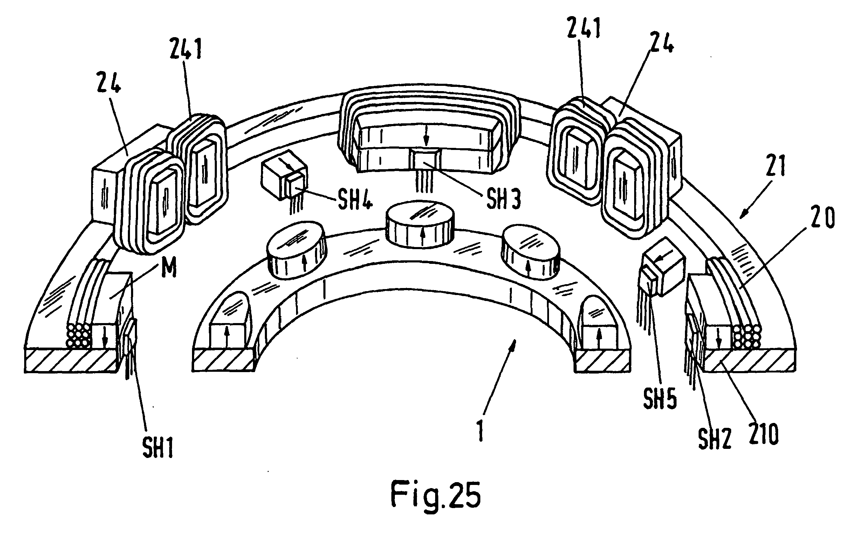

Insbesondere auf die Sensorik bei Anwendungen mit einem zwischen dem Stator und dem Rotor angeordneten Gefäss (z.B. das Pumpengehäuse 3 in Fig. 24) soll aber noch einmal eingegangen werden, weil bei der Verwendung bestimmter Materialien (z.B. Chromstahl) für das Gefäss (bzw. für das Pumpengehäuse) die sonst typischerweise für die Bestimmung der Position des Rotors 1 eingesetzten Wirbelstromsensoren relativ schnell an ihre Grenzen stossen. Hier erweisen sich magnetische Sensoren, insbesondere die bereits erwähnten Hall-Sensoren, als besonders vorteilhaft. Wie solche Hall-Sensoren angeordnet sein können, ist in dem Ausführungsbeispiel gemäss Fig. 25 gezeigt. Das dort dargestellte Ausführungsbeispiel entspricht im wesentlichen dem Ausführungsbeispiel gemäss Fig. 14 (Segmentanordnung), allerdings sind hier die Sensoren anders angeordnet bzw. ausgebildet.

Grundsätzlich können die Hall-Sensoren im Luftspalt zwischen dem Lagerstator 21 und dem Rotor 1 angeordnet sein. Sie können aber insbesondere auch auf den Zähnen 210 des Lagerstators 21 befestigt (z.B. aufgeklebt) sein, wie dies die Hall-Sensoren SH1,SH2,SH3 sind. Alternativ können sie auch an anderer Stelle im Luftspalt angeordnet werden, wie dies die Hall-Sensoren SH4,SH5 sind. In diesem Fall sind die Hall-Sensoren auf einem separaten Permanentmagneten angeordnet. Bei beiden Varianten werden also die Hall-Sensoren von einer relativ grossen Ruhedurchflutung (bei der aufgeklebten Variante wird diese von den Permanentmagneten im Stator erzeugt, bei der anderen Variante von dem direkt am Sensor befindlichen separaten Permanentmagneten) durchsetzt, sodass der mögliche Einfluss von Streufeldern, die von den Spulenkernen 24 und deren Wicklungen 241 herrühren, gering ist. Ohne Ruhedurchflutung würden sonst auch durch die vorbeiwandernden Permanentmagnete vergleichsweise hohe Flusschwankungen auftreten. Zur Bestimmung der Position des Rotors in der jeweiligen Richtung eignet sich die Differenz der Signale von zwei geometrisch gegenüberliegenden Sensoren besonders gut, auch wenn vom Prinzip her ein Sensor je Richtung ausreicht.

**Patentansprüche**

1. Magnetgelagerte Rotationsanordnung mit einem scheibenförmigen oder ringförmigen magnetisch gelagerten Rotor (1), umfassend einen aus einem ferromagnetischen Material bestehenden Ring (10), und Permanentmagneten (M), die in Umfangsrichtung verteilt auf dem scheiben- oder ringförmigen Rotor (1) angeordnet sind und einen örtlich modulierten unipolaren Vormagnetisierungsfluss bewirken und mit einem Stator (2), der Mittel zur Erzeugung eines mit dem örtlich modulierten unipolaren Vormagnetisierungsfluss wechselwirkenden Feldes umfasst, das eine Drehung des Rotors (1) bewirkt und im Stator (2) Permanentmagnete (M) vorgesehen sind, die mit den am Rotor vorgesehenen Permanentmagneten (M) derart zusammenwirken, dass sie die magnetische Lagerung des Rotors bewirken bzw. verstärken, wobei die im Rotor (1) angeordneten Permanentmagnete (M) auf einer oberen Fläche und / oder einer unteren Fläche des aus einem ferromagnetischem Material bestehenden Rings (10) angeordnet sind.

2. Rotationsanordnung nach Anspruch 1, **dadurch gekennzeichnet, dass** die Permanentmagnete (M) des Rotors (1) beidseitig des scheiben- oder ringförmigen Rotors angeordnet sind.

3. Rotationsandordnung nach einem der Ansprüche 1 oder 2, **dadurch gekennzeichnet, dass** die Permanentmagnete (M) des Rotors eine axiale oder radiale Magnetisierung aufweisen.

4. Rotationsanordnung nach einem der vorangehenden Ansprüche, **dadurch gekennzeichnet, dass** sowohl die auf dem Rotor (1) vorgesehenen Permanentmagnete (M) als auch die im Stator (2) angeordneten Permanentmagnete (M) in axialer Richtung magnetisiert sind.

5. Rotationsanordnung nach einem der Ansprüche 1 bis 3, **dadurch gekennzeichnet, dass** sowohl die auf dem Rotor (1) vorgesehenen Permanentmagnete (M) als auch die im Stator (2) angeordneten Permanentmagnete (M) in radialer Richtung magnetisiert sind.

6. Rotationsanordnung nach einem der Ansprüche 1 bis 3, **dadurch gekennzeichnet, dass** die auf dem Rotor (1) vorgesehenen Permanentmagnete (M) in axialer Richtung magnetisiert sind, während die im Stator (2) angeordneten Permanentmagnete (M) in radialer Richtung magnetisiert sind, oder umgekehrt.

7. Rotationsanordnung nach einem der vorangehenden Ansprüche, **dadurch gekennzeichnet, dass** im Stator (2) Steuerwicklungen (20) vorgesehen sind, um den örtlich modulierten unipolaren Vormagnetisierungsfluss zu steuern.

8. Rotationsanordnung nach einem der Ansprüche 1 bis 6, **dadurch gekennzeichnet, dass** der die magnetische Lagerung des Rotors bewirkende Stator (2,21) ringförmig ausgebildet ist und den ring- oder scheibenförmigen Rotor umschliesst, wobei eine Statorebene, deren Flächennormale parallel zu einer Flächennormalen des ring- oder scheibenförmigen Rotors liegt, und eine Rotorebene, deren Flächennormale parallel zu der Flächennormalen des ring- oder scheibenförmigen Rotors liegt und die Rotorebene zwischen der oberen Fläche und der unteren Fläche des ferromagnetischen Rings des Rotors liegt, zusammenfallen und eine Lagerebene bilden, deren Flächennormale parallel zu der Flächennormalen des ring oder scheibenförmigen Rotors liegt, und dass die Mittel zur Erzeugung des Felds, welches die Drehung des Rotors (1) bewirkt, in einem als Segment ausgestalteten Bereich zwischen zwei zueinander beabstandeten Permanentmagneten (M) im Stator (2,21) angeordnet sind, sodass die Motorebene, in der die Drehung des Rotors (1) bewirkt wird und deren Flächennormale parallel zu der Flächennormalen des ring- oder scheibenförmigen Rotors liegt, und die Lagerebene, in der die Lagerung des Rotors bewirkt wird, zusammenfallen.

9. Rotationsanordnung nach Anspruch 8, **dadurch gekennzeichnet, dass** die in den Segmenten zwischen den Permanentmagneten (M) angeordneten Mittel zur Erzeugung des Felds, welches die Drehung des Rotors bewirkt, U-förmige Spulenkerne (24) mit Wicklungen (241) aufweist, wobei die U-förmigen Spulenkerne (24) in der Lagerebene angeordnet sind.

10. Rotationsanordnung nach Anspruch 8, **dadurch gekennzeichnet, dass** die in den Segmenten zwischen den Permanentmagenten (M) angeordneten Mittel zur Erzeugung des Felds, welches die Drehung des Rotors (1) bewirkt, U-förmige Spulenkerne (24) mit Wicklungen (241) aufweist, wobei die U-förmigen Spulenkerne (24) senkrecht zur Lagerebene angeordnet sind.

11. Rotationsanordnung nach einem der Ansprüche 1 bis 6, **dadurch gekennzeichnet, dass** der die magnetische Lagerung des Rotors bewirkende Stator (2,21) ringförmig ausgebildet ist und den ring- oder scheibenförmigen Rotor (1) umschliesst, wobei eine Statorebene, deren Flächennormale parallel zu einer Flächennormalen des ring- oder scheibenförmigen Rotors liegt, und eine Rotorebene, deren Flächennormale parallel zu der Flächennormalen des ring- oder scheibenförmigen Rotors liegt, zusammenfallen und eine Lagerebene bilden, deren Flächennormale parallel zu der Flächennormalen des ring- oder scheibenförmigen Rotors liegt, und dass der Stator darüberhinaus noch mindestens einen weiteren ring- oder scheibenförmigen Motorstator (22,23) umfasst, der in einer Motorebene, deren Flächennormale parallel zu der Flächennormalen des ring- oder scheibenförmigen Rotors liegt, parallel zur Lagerebene angeordnet ist.

**12.** Rotationsanordnung nach Anspruch 11, **dadurch gekennzeichnet, dass** die Permanentmagnete (M) beidseitig auf dem Rotor (1) angeordnet sind, und dass der Stator zusätzlich zu dem die magnetische Lagerung des Rotors bewirkenden ringförmigen Stator (21) zwei weitere ringförmige Motorstatoren (22,23) umfasst, von denen ein Motorstator (22) in einer zur Lagerebene parallelen ersten Motorebene auf der einen Seite des Lagerstators und der andere (23) in einer zweiten Motorebene parallel zur Lagerebene angeordnet sind.

**13.** Rotationsanordnung nach Anspruch 11, **dadurch gekennzeichnet, dass** der Stator zusätzlich zu dem die magnetische Lagerung des Rotors (1) bewirkenden ringförmigen Stator (21) einen weiteren scheibenförmigen Motorstator in einer zur Lagerebene parallelen Motorebene umfasst, wobei dieser Motorstator (25) als Scheibenläuferstator ausgebildet ist und eine vorzugsweise eisenlose Scheibenläufer-Wicklung (250) zur Erzeugung des Felds für die Drehung des Rotors (1) aufweist.

**14.** Rotationsanordnung nach einem der Ansprüche 1 bis 6, **dadurch gekennzeichnet, dass** die Mittel zur Erzeugung des Felds, welches die Drehung des Rotors (1) bewirkt, einen rotierbaren, magnetisch mit dem Rotor (1) koppelbaren Antrieb (26) umfassen, dessen Drehachse mit der Drehachse des Rotors (1) zusammenfällt.

**15.** Rotationsanordnung nach Anspruch 14, **dadurch gekennzeichnet, dass** der Antrieb (26) in axialer Richtung magnetisierte Permanentmagnete (M) umfasst.

**16.** Rotationsanordnung nach Anspruch 14, **dadurch gekennzeichnet, dass** der Antrieb (26) in radialer Richtung magnetisierte Permanentmagnete (M) umfasst.

**17.** Fördervorrichtung, insbesondere für hochreine oder biologische Flüssigkeiten, speziell Blutpumpe, mit einer Rotationsanordnung gemäss einem der Ansprüche 1 bis 16.

**18.** Rührwerk für einen Bioreaktor, mit einer Rotationsanordnung gemäss einem der Ansprüche 1 bis 16.

## Claims

**1.** A magnetically journalled rotational arrangement with a disc-shaped or ring-shaped magnetically journalled rotor (1) including a ring (10) consisting of a ferromagnetic material and permanent magnets (M) which are arranged distributed in the peripheral direction on the disc-shaped or ring-shaped rotor (1) and which bring about a locally modulated unipolar pre-magnetisation flux and with a stator (2) which includes means for producing a field which interacts with the locally modulated unipolar pre-magnetisation flux, the field bringing about a rotation of the rotor (1) and with permanent magnets (M) being provided in the stator (2) which cooperate with the permanent magnets (M) provided at the rotor in such a way that they bring about or amplify the magnetic support of the rotor, wherein the permanent magnets (M) arranged in the rotor (1) are disposed on an upper surface and/or on a lower surface of the ring (10) consisting of ferromagnetic material.

**2.** A rotational arrangement in accordance with claim 1 **characterised in that** the permanent magnets (M) of the rotor are arranged at both sides of the disc-shaped or ring-shaped rotor.

**3.** A rotational arrangement in accordance with claim 1 or claim 2 **characterised in that** the permanent magnets (M) of the rotor have an axial or a radial magnetisation.

**4.** A rotational arrangement in accordance with any one of the preceding claims **characterised in that** both the permanent magnets (M) provided on the rotor (1) and the permanent magnets (M) arranged in the stator (2) are magnetised in the axial direction.

**5.** A rotational arrangement in accordance with any one of the claims 1 to 3 **characterised in that** both the permanent magnets (M) provided on the rotor (1) and the permanent magnets (M) arranged in the stator (2) are magnetised in the radial direction.

**6.** A rotational arrangement in accordance with any one of the claims 1 to 3 **characterised in that** the permanent magnets (M) provided on the rotor (1) are magnetised in the axial direction while the permanent magnets (M) arranged in the stator (2) are magnetised in the radial direction or vice versa.

**7.** A rotational arrangement in accordance with any one of the preceding claims **characterised in that** control windings (20) are provided in the stator (2) in order to control the locally modulated unipolar pre-magnetisation flux.

8. A rotational arrangement in accordance with any one of the claims 1 to 6, **characterised in that** the stator (2, 21) which brings about the magnetic journaling of the rotor is of ring-like shape and surrounds the ring-shaped or disc-shaped rotor, wherein a stator plane - whose normal to the surface lies parallel to a normal to the surface of the ring-shaped or disc-shaped rotor and a rotor plane - whose normal to the surface lies parallel to the normal to the surface of the ring-shaped or disc-shaped rotor and which lies between the upper surface and the lower surface of the ferromagnetic ring of the rotor - coincide and form a support plane whose normal to the surface lies parallel to the normal to the surface of the ring-shaped or disc-shaped rotor and **in that** the means for producing the field which brings about the rotation of the rotor (1) are arranged in a region formed as a segment between two mutually spaced apart permanent magnets (M) in the stator (2, 21), so that the motor plane in which the rotation of the rotor (1) is brought about and the support plane in which the support of the rotor is brought about coincide, with the normal to the surface of motor plane lying parallel to the normal to the surface of the ring-shaped or disc-shaped rotor.

9. A rotational arrangement in accordance with claim 8, **characterised in that** the means for the generation of the field which brings about the rotation of the rotor which are arranged in the segments between the permanent magnets (M) have U-shaped coil cores (24) with windings (241), with the U-shaped coil cores (24) being disposed in the support plane.

10. A rotational arrangement in accordance with claim 8, **characterised in that** the means for the generation of the field which brings about the rotation of the rotor which are arranged in the segments between the permanent magnet (M) have U-shaped coil cores (24) with windings (241), with the U-shaped coil cores (24) being disposed perpendicular to the support plane.

11. A rotational arrangement in accordance with any one of the claims 1 to 6, **characterised in that** the stator (2, 21) which brings about the magnetic support of the rotor is of ring-like shape and surrounds the ring-shaped or disc-shaped rotor (1), wherein a stator plane - whose normal to the surface lies parallel to a normal to the surface of the ring-shaped or disc-shaped rotor - and a rotor plane - whose normal to the surface lies parallel to the normal to the surface of the ring-shaped or disc-shaped rotor - coincide and form a support plane whose normal to the surface lies parallel to the normal to the surface of the ring-shaped or disc-shaped rotor ; and **in that** the stator in addition includes at least one further ring-shaped or disc-shaped motor stator (22, 23) which is disposed parallel to the support plane in a motor plane whose normal to the surface lies parallel to the normal to the surface of the ring-shaped or disc-shaped rotor.

12. A rotational arrangement in accordance with claim 11, **characterised in that** the permanent magnets (M) are arranged on the rotor (1) at both sides and **in that** the stator includes, in addition to the ring-like stator (21) which brings about the magnetic support of the rotor, two further ring-like motor stators (22, 23) of which one motor stator (22) is arranged in a support plane parallel to the first motor plane at the one side of the support stator and the other (23) is arranged in a second motor plane parallel to the support plane.

13. A rotational arrangement in accordance with claim 11, **characterised in that** the stator includes, in addition to the ring-like stator (21) which brings about the magnetic support of the rotor (1) a further disc-shaped motor stator in a motor plane parallel to the support plane, with this motor stator (25) being formed as a disc armature stator and having a preferably iron-free disc armature winding (250) for generating the field for the rotation of the rotor (1).

14. A rotational arrangement in accordance with any one of the claims 1 to 6, **characterised in that** the means for the generating of the field which brings about the rotation of the rotor (1) includes a rotatable drive (26) which can be magnetically coupled to the rotor (1), with the axis of rotation of the drive coinciding with the axis of rotation of the rotor (1).

15. A rotational arrangement in accordance with claim 14, **characterised in that** the drive (26) includes permanent magnets (M) magnetised in the axial direction.

16. A rotational arrangement in accordance with claim 14, **characterised in that** the drive (26) includes permanent magnets (M) magnetised in the radial direction.

17. A conveyor apparatus, in particular for high purity or biological liquids, especially a blood pump, having a rotational arrangement in accordance with any one of the claims 1 to 16.

18. A stirrer for a bioreactor having a rotational arrangement in accordance with any one of the claims 1 to 16.

## Revendications

1. Dispositif rotatif avec palier magnétique comportant un rotor (1) en forme de disque ou d'anneau avec palier magnétique, comportant un anneau (10) fabriqué dans un matériau ferromagnétique et des aimants permanents (M) qui sont répartis dans le sens circonférentiel sur le rotor (1) en forme de disque ou d'anneau et produisent un flux de prémagnétisation unipolaire modulé localement, et comportant un stator (2) qui comprend des dispositif de production d'un champ interagissant avec le flux de prémagnétisation unipolaire modulé localement, champ qui induit une rotation du rotor (1) et des aimants permanents (M) sont prévus dans le stator (2), aimants qui interagissent avec les aimants permanents (M) prévus sur le rotor de telle sorte qu'ils réalisent ou renforcent le logement magnétique du rotor, les aimants permanents (M) disposés dans le rotor (1) étant disposés sur une surface supérieure et/ou une surface inférieure de l'anneau (10) fabriqué dans un matériau ferromagnétique.

2. Dispositif rotatif selon la revendication 1, **caractérisé en ce que** les aimants permanents (M) du rotor (1) sont disposés des deux côtés du rotor en forme de disque ou d'anneau.

3. Dispositif rotatif selon l'une quelconque des revendications 1 ou 2, **caractérisé en ce que** les aimants permanents (M) du rotor présentent une magnétisation axiale ou radiale.

4. Dispositif rotatif selon l'une quelconque des revendications précédentes, **caractérisé en ce qu'**à la fois les aimants permanents (M) prévus sur le rotor (1) et les aimants permanents (M) disposés dans le stator (2) sont magnétisés dans le sens axial.

5. Dispositif rotatif selon l'une quelconque des revendications 1 à 3, **caractérisé en ce qu'**à la fois les aimants permanents (M) prévus sur le rotor (1) et les aimants permanents (M) disposés dans le stator (2) sont magnétisés dans le sens radial.

6. Dispositif rotatif selon l'une quelconque des revendications 1 à 3, **caractérisé en ce que** les aimants permanents (M) prévus sur le rotor (1) sont magnétisés dans le sens axial tandis que les aimants permanents (M) disposés dans le stator (2) sont magnétisés dans le sens radial ou inversement.

7. Dispositif rotatif selon l'une quelconque des revendications précédentes, **caractérisé en ce que** des enroulements de commande (20) sont prévus dans le stator (2) pour commander le flux de prémagnétisation unipolaire modulé localement.

8. Dispositif rotatif selon l'une quelconque des revendications 1 à 6, **caractérisé en ce que** le stator (2, 21) induisant le logement magnétique du rotor est réalisé sous forme d'anneau et entoure le rotor en forme d'anneau ou de disque, un plan de stator, dont la normale à une surface est parallèle à une normale à une surface du rotor en forme d'anneau ou de disque, et un plan de rotor, dont la normale à une surface est parallèle à la normale à une surface du rotor en forme d'anneau ou de disque, et le plan de rotor se trouvant entre la surface supérieure et la surface inférieure de l'anneau ferromagnétique du rotor, coïncident et forment un plan de palier dont la normale à une surface est parallèle à la normale à une surface du rotor en forme d'anneau ou de disque et **en ce que** les dispositifs de production du champ, lequel induit la rotation du rotor (1), sont disposés dans le stator (2, 21), dans une zone réalisée sous la forme de segment, entre deux aimants permanents (M) espacés l'un de l'autre, de telle sorte que le plan de moteur, sur lequel est induite la rotation du rotor (1) et dont la normale à une surface est parallèle à la normale à une surface du rotor en forme d'anneau ou de disque, et le plan de palier, sur lequel le logement du rotor est induit, coïncident.

9. Dispositif rotatif selon la revendication 8, **caractérisé en ce que** les dispositifs de production du champ, lequel induit la rotation du rotor, disposés dans les segments entre les aimants permanents (M), comportent des noyaux de bobine en U (24) avec des enroulements (241), les noyaux de bobine en U (24) étant disposés sur le plan de palier.

10. Dispositif rotatif selon la revendication 8, **caractérisé en ce que** les dispositifs de production du champ, lequel induit la rotation du rotor (1), disposés dans les segments entre les aimants permanents (M), comportent des noyaux de bobine en U (24) avec des enroulements (241), les noyaux de bobine en U (24) étant disposés perpendiculairement au plan de palier.

11. Dispositif rotatif selon l'une quelconque des revendications 1 à 6, **caractérisé en ce que** le stator (2, 21) induisant le logement magnétique du rotor est réalisé en forme d'anneau et entoure le rotor (1) en forme d'anneau ou de disque, un plan de stator, dont la normale à une surface est parallèle à une normale à une surface du rotor en forme d'anneau ou en forme de disque, et un plan de rotor, dont la normale à une surface est parallèle à la normale à une surface du rotor en forme d'anneau ou en forme de disque, coïncident et forment un plan de palier dont la normale à une surface est parallèle à la normale à une surface du rotor en forme d'anneau ou de disque, et **en ce que** le stator comporte en outre encore au moins un autre stator de moteur (22, 23) en forme d'anneau ou de disque qui est disposé sur un plan de moteur, dont la normale à une surface est parallèle à la normale à une surface du rotor en forme d'anneau ou de disque, de manière parallèle au plan de palier.

**12.** Dispositif rotatif selon la revendication 11, **caractérisé en ce que** les aimants permanents (M) sont disposés des deux côtés sur le rotor (1) et **en ce que** le stator comprend, en plus du stator annulaire (21) induisant le logement magnétique du rotor, deux autres stators de moteur annulaires (22, 23) parmi lesquels un stator de moteur (22) est disposé sur un premier plan de moteur parallèle au plan de palier sur un côté du stator à palier et l'autre (23) sur un second plan de moteur parallèle au plan de palier.

**13.** Dispositif rotatif selon la revendication 11, **caractérisé en ce que** le stator comprend, en plus du stator annulaire (21) induisant le logement magnétique du rotor (1), un autre stator de moteur en forme de disque sur un plan de moteur parallèle au plan de palier, ce stator de moteur (25) étant réalisé sous la forme de stator avec induit à disque et comportant un enroulement avec induit à disque (250) sans fer de préférence pour la production du champ servant à la rotation du rotor (1).

**14.** Dispositif rotatif selon l'une quelconque des revendications 1 à 6, **caractérisé en ce que** les dispositifs de production du champ, lequel induit la rotation du rotor (1), comportent un entraînement (26) rotatoire, pouvant être couplé de manière magnétique au rotor (1), entraînement dont l'axe de rotation coïncide avec l'axe de rotation du rotor (1).

**15.** Dispositif rotatif selon la revendication 14, **caractérisé en ce que** l'entraînement (26) comporte des aimants permanents (M) magnétisés dans le sens axial.

**16.** Dispositif rotatif selon la revendication 14, **caractérisé en ce que** l'entraînement (26) comporte des aimants permanents (M) magnétisés dans le sens radial.

**17.** Dispositif d'acheminement, en particulier pour des liquides ultra-purs ou biologiques, une pompe à sang en particulier, comportant un dispositif rotatif selon l'une quelconque des revendications 1 à 16.

**18.** Mélangeur pour un bioréacteur comportant un dispositif rotatif selon l'une quelconque des revendications 1 à 16.

Fig.1

Fig.2

Fig.3

Fig.4

Fig.5

Fig.6

# Fig.7

Fig.8

Fig.9

Fig.10

Fig.11

**Fig.12**

**Fig.13**

## Fig.14

## Fig.15

17

# Fig.16

# Fig.17

## Fig.18

## Fig.19

Fig.20

Fig.21

Fig.22

## Fig.23

## Fig.24

Fig.25